(19) Europäisches Patentamt / European Patent Office / Office européen des brevets

(11) EP 2 935 371 B1

(12) **EUROPEAN PATENT SPECIFICATION**

(45) Date of publication and mention of the grant of the patent:
**11.07.2018 Bulletin 2018/28**

(21) Application number: **13811505.0**

(22) Date of filing: **19.12.2013**

(51) Int Cl.:
*C08F 220/06* (2006.01)     *C08F 220/54* (2006.01)
*C08F 220/12* (2006.01)     *A61Q 5/06* (2006.01)

(86) International application number:
**PCT/EP2013/077284**

(87) International publication number:
**WO 2014/096128 (26.06.2014 Gazette 2014/26)**

(54) **HAIR FIXATIVE COMPOSITIONS**

HAARFESTIGERZUSAMMENSETZUNGEN

COMPOSITIONS DE FIXATION DE CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **21.12.2012  US 201261740736 P**
**08.03.2013  EP 13158431**

(43) Date of publication of application:
**28.10.2015  Bulletin 2015/44**

(73) Proprietor: **Akzo Nobel Chemicals International B.V.**
**6824 BM Arnhem (NL)**

(72) Inventors:
• **PHILBIN, Michael, Timothy**
**Hopewell, New Jersey 08525 (US)**
• **BELLUSCIO, Maryalice**
**Long Valley, New Jersey 07853 (US)**
• **HE, Qiwei**
**Belle Mead, New Jersey 08502 (US)**

(74) Representative: **Akzo Nobel Chemicals IP Group**
**Velperweg 76**
**6824 BM Arnhem (NL)**

(56) References cited:
**WO-A1-94/02112     US-B1- 6 482 393**

**Description**

FIELD OF THE INVENTION

The present invention relates to hair fixative compositions. More specifically, the present invention relates to hair fixative compositions comprising at least one polymer derived from at least one acid-containing monomer and at least one N-alkyl (meth)acrylamide monomer.

BACKGROUND OF THE INVENTION

Governmental agencies, such as the California Air Resource Board, are currently considering using the Maximum Incremental Reactivity (MIR) scale to rank the ozone-forming potential of all volatile organic compounds (VOCs). Consequently, the current consumer product regulation for hair fixatives containing VOCs, such as hairsprays, would be modified from the current VOC scale to the MIR scale. Although no MIR Value for hairsprays has yet been adopted, some proposals would require hairspray compositions to have a MIR value of less than 0.80. Accordingly, if an MIR of 0.80 or less is implemented, reformulation of current anhydrous 55% VOC aerosol hairsprays and all non-aerosol hairsprays would be required to comply with the new regulations. Accordingly, there is a need for hair fixative polymers that are soluble in surfactant systems, such as ethanol, isopropanol, water and mixtures thereof and that also provide low viscosity and low turbidity. Thus, the hair fixative formulations that include these polymers must not only meet the more stringent MIR requirements, but they must meet or exceed the performance of conventional hair fixative formulations.

SUMMARY OF THE INVENTION

In an aspect, , the present invention relates to a hair fixative composition comprising at least one fixative polymer derived from at least one acid-containing monomer, at least one N-alkyl (meth)acrylamide and, optionally, an alkyl (meth)acrylate, a solvent system, and, optionally, a propellant.

DETAILED DESCRIPTION OF THE INVENTION

The present invention generally relates to hair fixative formulations that include at least one film forming polymer that is soluble in a solvent system such that the hair fixative composition has a MIR value of about 0.80 or less. The hair fixative formulations provide low viscosity and low turbidity. The present invention relates to a hair fixative formulation comprising a polymer derived from 18 to 25 weight percent of at least one acid-containing monomer and at least one N-alkyl (meth)acrylamide monomer that is N-n-octyl acrylamide or N-t-octyl acrylamide; and a solvent system comprising one or more solvents selected from the group consisting of a C2-C6 straight or branched chain alcohol, butyl cellusolve, propylene glycol, water and mixtures thereof, with the proviso that the at least one fixative polymer is solution polymerized such that the fixative polymer is a solute dissolved in the solvent system.. The hair fixative formulation optionally comprises at least one alkyl (meth)acrylate.

The hair fixative polymers contain at least one acid-containing monomer. Some non-limiting examples of these acid-containing monomers include maleic acid, fumaric acid, acrylic acid, methacrylic acid, itaconic acid. Also suitable are $C_1$ -$C_4$ alkyl half esters of maleic and fumaric acids such as methyl hydrogen maleate and butyl hydrogen fumarate, as well as any other acidic monomers which are capable of being copolymerized with the particularly desired polymeric binder system. As is known to those of ordinary skill in the art, the acidic co-monomer(s) must be chosen so that they are readily polymerizable with the polymer. In an embodiment, the acid-containing monomer(s) are acrylic acid, methacrylic acid, crotonic acid, itaconic acid and maleic acid. Mixtures of the various above-described monomers may also be used. In a preferred embodiment, the acid-containing monomer is acrylic acid or methacrylic acid.

The acid-containing monomer is present from about 18 to about 25 weight percent based on total monomer content in the dry polymer, and in another embodiment about 18 to about 21 percent.

The hair fixative polymers contain at least one N-alkyl (meth)acrylamide monomer that is N-n-octyl acrylamide or N-t-octyl acrylamide.

In an embodiment, the amount of N-alkyl (meth)acrylamides present in the polymers of the current invention may be from about 19 percent to about 84 percent by weight based on total weight of the dry polymer. In another embodiment, the N-alkyl (meth)acrylamides are present from about 5 percent to about 30 percent of the total dry weight of the polymer. In yet another embodiment, the N-alkyl (meth)acrylamides are present from about 19 percent to about 30 percent of the total dry weight of the polymer. In still another embodiment, the N-alkyl (meth)acrylamide may be present from about 60 to about 90 percent of the total monomer content in the dry polymer. In still yet another embodiment, the N-alkyl (meth)acrylamide may be present from about 60 to about 85 percent of the total monomer content in the dry polymer.

In another embodiment, it may be desirable to have an optional alkyl (meth)acrylate monomer included into the polymers of this invention. The alkyl group of the alkyl (meth)acrylate may contain from about 1 to about 8 carbon atoms and are uncharged (i.e. nonionic). Some non-limiting examples of suitable alkyl (meth)acrylates include methyl acrylate, butyl acrylate, ethyl acrylate, methyl methacrylate, ethyl methacrylate, n-butyl methacrylate, iso-butyl methacrylate and n-octyl methacrylate as well as mixtures thereof. In an embodiment, the alkyl acrylate may be n-butyl acrylate, iso-butyl acrylate, n-butyl methacrylate, iso-butyl methacrylate or n-octyl methacrylate.

In an embodiment, the alkyl (meth)acrylate monomers may be present in the amount up to about 60 percent by weight of the total monomer content in the dry polymer. In another embodiment, the alkyl (meth)acrylate will be present up to about 40 percent by weight of the monomer content in dry polymer. In yet another embodiment the alkyl (meth)acrylate is present from about 1 to about 60 percent by weight of the total monomer content in the polymer. In still yet another embodiment, the alkyl (meth)acrylate is present from about 1 to about 40 percent of the total monomer content in the dry polymer, and in another embodiment about 10 to about 35 percent. In even still yet another embodiment, the alkyl (meth)acrylate is present from about 55 to about 60 percent of the total monomer content in the dry polymer.

In an embodiment, the hair fixative polymer is present in the hair fixative composition in an amount of from 0.25% to 8.0% by weight of the composition. In another embodiment, the hair fixative polymer is present in the hair fixative composition in an amount of fro 2.0% to 8.0% by weight of the composition. In another embodiment, the hair fixative polymer is present in the hair fixative composition in an amount of from 1.0% to 6.0% by weight of the composition.

In another aspect, solvent systems suitable for use in the present invention comprise at least one solvent that is chosen from a $C_2$-$C_6$ straight or branched chain alcohol, butyl cellusolve, propylene glycol or water or mixtures thereof, such that the hair fixative composition has a MIR value of about 0.80 or less.

In an embodiment of this invention the solvent system is a mixture of ethanol and isopropanol in a weight ratio of 80:20 to 20:80. In a further embodiment, the weight ratio is from about 75:25 to about 25:75. In yet another embodiment, the weight ratio is from about 70:30 to about 30:70, in a further embodiment a weight ratio of about 65:35 to about 35:65, and in still yet another embodiment a weight ratio of about 60:40 to about 40:60. In another embodiment, the ratios are from greater than 50:50 to about 75:25.

In another embodiment the solvent system that is a mixture of ethanol/isopropanol mixture further contains at most about 50% water, in another embodiment at most about 40% water and in yet another embodiment at most about 25% water, while still maintaining the ratio of ethanol to isopropanol. In an embodiment, the solvent system contains about 1% or more water, in another embodiment about 5% or more water and in yet another embodiment about 10% or more water.

In another embodiment, the solvent system comprises one or more non-aqueous solvents that is not an acetate, such as methyl or ethyl acetate, or a ketone, such as acetone or methyl ethyl ketone.

The hair fixative polymers of the present invention provide low turbidity in the solvent system. The turbidity of a 5% solution of the polymer that has had 100% of the acid groups of the acid-containing monomer neutralized with 2-Amino-2-methyl-1-Propanol will be less than 100 NTUs in the solvent system (not including any propellants). In an embodiment of this invention, the turbidity of the polymers will be about 50 or less, particularly about 25 or less.

In an embodiment, the polymers of the present invention also provide low viscosity to the hair fixative compositions. Having a low viscosity allows the polymer solution to be sprayed from solution and provides uniform coverage and small droplet size. Therefore, the polymers of the present invention provide low viscosity (cps) at 5 % polymer concentration, at 25°C and neutralized 100% with 2-Amino-2-methyl-1-Propanol in the solvent system (not including any propellants). For purposes of the invention, in an embodiment, low viscosity refers to viscosities of less than about 12 cps. In another embodiment, the viscosity of the polymers in the solvent system will be less than about 10, particularly less than about 5 cps at 25°C and at 5% polymer solids.

For purposes of this invention, propellants are not included as part of the solvent system as defined herein, although one or more propellants may optionally be included as components of the hair fixative composition. The aerosol propellants suitable for use in the present invention include, but are not limited to, Such propellants include, without limitation, ethers, such as dimethyl ether; one or more lower boiling hydrocarbons such as $C_3$-$C_6$ straight and branched chain hydrocarbons, for example, propane, butane, and isobutane; halogenated hydrocarbons, such as, hydrofluorocarbons, for example, trichlorofluoromethane , dichlorodifluoromethane, 1,1-difluoroethane and 1,1,1,2-tetrafluoroethane, present as a liquefied gas; and the compressed gases, for example, nitrogen, air and carbon dioxide and mixtures of the various propellants. In an embodiment, the propellant is present in an amount from about 10 to about 85 % by weight based on total weight of the composition. In a further embodiment, the propellant is present in an amount of about 20% to about 75 % by weight. In a further embodiment, the propellant is present in an amount of about 25% to about 55 % by weight.

In an embodiment, the solvent system used in the hair fixative composition comprises about 8% to about 95% by weight of the hair fixative composition, and in another embodiment, about 20% to about 70% by weight. In another embodiment, the solvent system used in the hair fixative composition comprises about 25% to about 55% by weight of the hair fixative composition. In another embodiment, the solvent system used in the hair fixative composition comprises about 40% to about 95% by weight of the hair fixative composition.

In addition to the solvent system, the hair fixative composition can also include an optional second fixative polymer. Non-limiting examples of these additional hair fixative polymers include: from Akzo Nobel Surface Chemistry LLC, AMPHOMER® 4961 and AMPHOMER® LV-71 polymers (octylacrylamide/acrylates/butylaminoethyl methacrylate compolymer), AMPHOMER® HC polymer (acrylates/octylacrylamide copolymer) BALANCE® 0/55 and BALANCE® CR polymers (acrylates copolymer), BALANCE® 47 polymer (octylacrylamide/butylaminoethyl methacrylate copolymer), RESYN® 28-2930 polymer (VA/crotonates/vinyl neodecanoate copolymer), RESYN® 28-1310 polymer (VA/Crotonates copolymer), FLEXAN® polymers (sodium polystyrene sulfonate), DynamX polymer (polyurethane-14 (and) AMP-Acrylates copolymer), RESYN® XP polymer (acrylates/octylacrylamide copolymer), STRUCTURE® 2001 (acrylates/steareth-20 itaconate copolymer) and STRUCTURE® 3001 (acrylates/ceteth-20 itaconate copolymer); from ISP, OMNIREZ-2000® (PVM/MA half ethyl ester copolymer), GANEX P-904® (butylated PVP), GANEX V-216® (PVP/hexadecene copolymer) GANEX® V-220 (PVP/eicosene copolymer), GANEX® WP-660 (tricontanyl PVP), GANTREZ® A425 (butyl ester of PVM/MA copolymer), GANTREZ® AN-119 PVM/MA copolymer, GANTREZ® ES 225 (ethyl ester of PVM/MA copolymer), GANTREZ® ES425 (butyl ester of PVM/MA copolymer), GAFFIX® VC-713 (vinyl caprolactam/PVP/dimethylaminoethyl methacrylate copolymer), GAFQUAT® 755 (polyquaternium-11), GAFQUAT HS-100® (polyquaternium-28) AQUAFLEX® XL-30 (Polyimide-1), AQUAFLEX® SF-40 (PVP/Vinylcaprolactam/DMAPA Acrylates Copolymer), AQUAFLEX® FX-64 (Isobutylene/Ethylmaleimide/Hydroxyethylmaleimide Copolymer), ALLIANZ® LT-120 (Acrylates/C1-2 Succinates/Hydroxyacrylates Copolymer), STYLEZE® CC-10 (PVP/DMAPA Acrylates Copolymer), STYLEZE® 2000 (VP/Acrylates/Lauryl Methacrylate Copolymer), STYLEZE® W-20 (Polyquaternium-55), Copolymer Series (PVP/Dimethylaminoethylmethacrylate Copolymer), ADVANTAGE® S and ADVANTAGE® LCA (VinylcaprolactamNP/Dimethylaminoethyl Methacrylate Copolymer), ADVANTAGE® PLUS (VA/Butyl Maleate/Isobornyl Acrylate Copolymer); from BASF, ULTRAHOLD STRONG (acrylic acid/ethyl acrylate/t-butyl acrylamide), LUVIMER® 100P (t-butyl acrylate/ethyl acrylate/methacrylic acid), LUVIMER® 36D (ethyl acrylate/t-butyl acrylate/methacrylic acid), LUVIQUAT® HM-552 (polyquaternium-16), LUVIQUAT® HOLD (polyquaternium-16), LUVISKOL® K30 (PVP) LUVISKOL® K90 (PVP), LUVISKOL® VA 64 (PVP/VA copolymer) LUVISKOL® VA73W (PVP/VA copolymer), LUVISKOL® VA, LUVISET® PUR (Polyurethane-1), LUVISET® Clear (VP/MethacrylamideNinyl Imidazole Copolymer), LUVIFLEX® SOFT (Acrylates Copolymer), ULTRAHOLD® 8 (Acrylates/Acrylamide Copolymer), LUVISKOL® Plus (Polyvinylcaprolactam), LUVIFLEX® Silk (PEG/PPG-25/25 Dimethicone/Acrylates Copolymer); from Amerchol, AMERHOLD® DR-25 (acrylic acid/methacrylic acidlacrylates/methacrylates); from Rohm&Haas, ACUDYNE® 258 (acrylic acid/methacrylic acid/acrylates/methacrylates/hydroxy ester acrylates; from Mitsubishi and distributed by Clariant, DIAFORMER® Z-301, DIAFORMER® Z-SM, and DIAFORMER® Z-400 (methacryloyl ethyl betaine/acrylates copolymer), ACUDYNE® 180 (Acrylates/Hydroxyesters Acrylates Copolymer), ACUDYNE® SCP (Ethylenecarboxyamide/AMPSA/Methacrylates Copolymer), and the ACCLTLYN® rheological modifiers; from ONDEO Nalco, FIXOMER® A-30 and FIXOMER® N-28 (INCI names: methacrylic acid/sodium acrylamidomethyl propane sulfonate copolymer); from Noveon, FIXATE® G-100 (AMP-Acrylates/Allyl Methacrylate Copolymer), FIXATE PLUS® (Polyacrylates-X), CARBOPOL® Ultrez 10 (Carbomer), CARBOPOL® Ultrez 20 (Acrylates/C10-30 Alkyl Acrylates Copolymer), AVALURE® AC series (Acrylates Copolymer), AVALURE® UR series (Polyurethane-2, Polyurethane-4, PPG-17/IPDI/DMPA Copolymer); polyethylene glycol; water-soluble acrylics; water-soluble polyesters; polyacrylamides; polyamines; polyquatemary amines; styrene maleic anhydride (SMA) resin; polyethylene amine; and other conventional polymer that is polar solvent soluble or that can be made soluble through neutralization with the appropriate base. The official chemical description of each of these chemical names can be found in the INCI dictionary or at the website (www.ctfa.org). In an embodiment of the invention, the hair fixative polymer is selected from octylacrylamide/Acrylates/Butylaminoethyl Methacrylate Copolymer, polyurethane-14(and)acrylates copolymer and VA/crotonates/vinyl neodecanoate. A combination of one or more of the above hair fixative polymers is also contemplated as within the scope of the present invention.

In an embodiment of the invention, the optional second fixative polymer may be present in the hair fixative composition in an amount of about 0.1 to 10% by weight based on total weight of the composition. In a further embodiment, the fixative polymer is present in an amount of about 1 to 10% by weight and in a further embodiment in an amount of about 1 to 7% by weight.

In an embodiment, the hair fixative composition includes about 4.0 wt% water or less, based on total weight of the hair fixative composition. In another embodiment, water is present in an amount of about 3.5 wt% or less, in yet another embodiment, water is present in an amount of about 3.0 wt% water or less, and still yet another embodiment, water is present in an amount of about 2.5 wt% or less, and in a further embodiment, the hair fixative composition is nonaqueous.

Another feature of the present invention is that the hair fixative composition has a Maximum Incremental Reactivity (MIR) of less than about 0.8 in an embodiment, and in further embodiments the MIR value is about 0.75 or less or about 0.70 or less.

MIR is defined as an incremental reactivity (IR) calculated for a volatile organic mixture where the emissions of $NO_x$ ($NO + NO_2$) have been adjusted to maximize the calculated MIR. IR can be determined by the following formula (I):

$$IR = \Delta[O_3]/\Delta[VOC] \qquad (I)$$

Thus, for a specified set of meteorological conditions, emissions, and initial concentrations, the incremental reactivity of an organic compound is the change in the peak ozone concentration, $\Delta[O_3]$, in grams, divided by an incremental change in the initial concentration and emissions of the organic compound $\Delta[VOC]$, in grams. For a given volatile organic ingredient, its MIR has been determined and the MIR values can be obtained, for example from The California Environmental Protection Agency Air Resources Board at www.arb.ca.gov/research/reactivity/reactivity.htm.

Thus, to determine overall MIR value of the hair fixative compositions of the invention, the following formula was used:

$$\text{Wtd MIR Ingredient} = \text{MIR x Weight Fraction Ing.} \qquad (II)$$

$$\text{PWMIR} = (\text{Wtd MIR})1 + (\text{Wtd MIR})2 + \ldots \ldots + (\text{Wtd MIR})n \qquad (III)$$

where Wtd MIR ingredient is the weighted MIR value for each ingredient and PWMIR is the sum of the weighted MIR values of the ingredients of the composition.

In an embodiment of the invention, the hair fixative compositions optionally further include a neutralizing agent. In an embodiment of the invention, the fixative polymer is generally at least about 80% neutralized. In another embodiment, the fixative polymer is at least about 90% neutralized, and in an even further embodiment, the fixative polymer is 100% neutralized. Suitable basic neutralizing agents compatible with the composition can be employed, even inorganic materials such as sodium or potassium hydroxide. Generally organic amines or alkanolamines are readily used for neutralization. In an embodiment, the neutralizing agents include, but are not limited to aminomethylpropanol, and di- methyl stearamine, sodium hydroxide, potassium hydroxide and triethanolamine.. Inorganic materials, such as sodium or potassium hydroxide, may also be used. In an embodiment of the invention, the neutralizing agent is an organic amine or alkanolamine.

Other optional additives to provide certain modifying properties to the composition include, but are not limited to, silicones and silicone derivatives; humectants; moisturizers; plasticizers, such as glycerine, glycol and phthalate esters and ethers; emollients, lubricants and penetrants, such as lanolin compounds; fragrances and perfumes; UV absorbers; dyes, pigments and other colorants; anticorrosion agents; antioxidants; detackifying agents; combing aids and conditioning agents; antistatic agents; neutralizers; glossifiers; proteins, protein derivatives and amino acids; vitamins; emulsifiers; surfactants; viscosity modifiers; stabilizers; sequestering agents; chelating agents; aesthetic enhancers; fatty acids, fatty alcohols and triglycerides; botanical extracts; film formers; and clarifying agents. Such additives are commonly used in hair cosmetic compositions known heretofore. These additives are present in small, effective amounts to accomplish their function, and generally will comprise from about 0.01 to 10% by weight each, and from about 0.01 to 20% by weight total, based on the weight of the composition.

The hair fixative compositions of the present invention may be, but are not limited, to aerosol and non-aerosol hairsprays.

The present invention is useful for providing low viscosity and turbidity to hair fixative formulations. In another aspect, the invention provides a method for preparing a hair fixative formulation comprising dissolving the least one fixative polymer in a solvent system wherein the solvent system comprises a solvent system comprising one or more solvents selected from the group consisting of a $C_2$-$C_6$ straight or branched chain alcohol, butyl cellusolve, propylene glycol, water and mixtures thereof, wherein the at least one polymer is derived from at least one acid-containing monomer and at least one N-alkyl (meth)acrylamide monomer.

In an embodiment, the method further includes neutralizing the solution with a neutralizing agent, such as aminomethylpropanol. In a further embodiment, the fixative polymer is dissolved in a first non-aqueous solvent, next the solution is neutralized and then a second non-aqueous solvent, and optionally water, is added to the solution. The first and second non-aqueous solvents may be present in the solvent system in a weight ratio of about 80:20 to about 20:80 of first non-aqueous solvent to second non-aqueous solvent. In an embodiment, the method further includes the steps of isolating the polymer in a solution, such as an i-Propyl Acetate/Ethanol solution, removing the solvent, such as by drying, and isolating the polymer in solid, for example powder, form.

The fixative polymers are solution polymerized such that the fixative polymers are solutes dissolved in the solvent system. In a further embodiment, the fixative polymers are not included in the hair fixative composition as an emulsion. In yet another embodiment, the hair fixative composition is not an aqueous emulsified hair fixative composition.

The invention will be further described by means of the following examples, which are not intended to limit the invention, as defined by the appended claims, in any manner. All weights discussed in the document are expressed in term of dry weight and based on the total weight of the polymer.

**PREPERATION OF POLYMERS**

EXAMPLE 1

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 24,0g of i-Butyl Methacrylate, 12.6g of Methacrylic Acid, and 39.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 136.0g of i-Butyl Methacrylate, 71.4g of Methacrylic Acid, and 221.0g of t-Octylacrylamide as a 60% solution in Ethanol was added over 3.5 hours using a 1000 mL addition funnel. After this addition was completed, the funnel was rinsed with 5,0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 2 - not in accordance with the present invention

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 24.0g of i-Butyl Methacrylate, 9.6g of Methacrylic Acid, and 44.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 136.0g of i-Butyl Methacrylate, 54.4g of Methacrylic Acid, and 249.3g of t-Octylacrylamide as a 60% solution in Ethanol was added over 3.5 hours using a 1000 mL addition funnel. After this addition was completed, the funnel was rinsed with 5,0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to relfux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 3 - not in accordance with the present invention

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 9.6g of Methacrylic Acid, and 84.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 54.4g of Methacrylic Acid, and 476.0g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to refux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 4

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 12.6g of i-Butyl Methacrylate, 11.1g of Methacrylic Acid, and 61.5g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 71.4g of i-Butyl Methacrylate, 62.9g of Methacrylic Acid, and 348.5g of t-Octylacrylamide as a 60% solution in Ethanol was added over 3.5 hours using a 1000 mL addition funnel. After this addition was completed, the funnel was rinsed with 5,0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 5

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 12.6g of Methacrylic Acid, and 79.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 71.4g of Methacrylic Acid, and 447.7g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 6

To a 2000mL reaction vessel equipped with an agitator, a heating mantle, a condenser, a 125 mL addition funnel, a 1000mL addition funnel, and a nitrogen purge was added 60.6g of i-Propyl Acetate. The stirring was turned on at a rate of 200 rpm, to the reaction was added 2.5g of Benzoyl Peroxide, 34.4g of i-Butyl Methacrylate, 14.4g of Methacrylic Acid, and 52.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 137.6g of i-Butyl Methacrylate, 57.6g of Methacrylic Acid, and 208.0g of t-Octylacrylamide as a 60% solution in Ethanol was added over 3.5 hours using a 1000 mL addition funnel. After this addition was completed, the funnel was rinsed with 4.0g of Ethanol, which was then added to the reaction. 2 hours and 15 minutes after the start of reflux, 1.1g of Benzoyl Peroxide dissolved in 84.0g of i-Propyl Acetate was added over 2 hours. After this addition was completed the funnel was rinsed with 4.0g of i-Propyl Acetate which was then added to the reaction. The reaction was then allowed to reflux for an additional 8.5 hours. The reaction was then diluted with 108.4g of i-Propyl Acetate and 25.2g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 7

To a 2000mL reaction vessel equipped with an agitator, a heating mantle, a condenser, a 125 mL addition funnel, a 1000mL addition funnel, and a nitrogen purge was added 60.6g of i-Propyl Acetate. The stirring was turned on at a rate of 200 rpm, to the reaction was added 2.5g of Benzoyl Peroxide, 26.4g of i-Butyl Methacrylate, 16.4g of Methacrylic Acid, and 62.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 105.6g of i-Butyl Methacrylate, 65.6g of Methacrylic Acid, and 248.0g of t-Octylacrylamide as a 60% solution in Ethanol was added over 3.5 hours using a 1000 mL addition funnel. After this addition was completed, the funnel was rinsed with 4.0g of Ethanol, which was then added to the reaction. 2 hours and 15 minutes after the start of reflux, 1.1g of Benzoyl Peroxide dissolved in 84.0g of i-Propyl Acetate was added over 2 hours. After this addition was completed the funnel was rinsed with 4.0g of i-Propyl Acetate which was then added to the reaction. The reaction was then allowed to reflux for an additional 8.5 hours. The reaction was then diluted with 108.4g of i-Propyl Acetate and 25.2g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 8

To a 2000mL reaction vessel equipped with an agitator, a heating mantle, a condenser, a 125 mL addition funnel, a 1000mL addition funnel, and a nitrogen purge was added 60.6g of i-Propyl Acetate. The stirring was turned on at a rate of 200 rpm, to the reaction was added 2.5g of Benzoyl Peroxide, 30.4g of i-Butyl Methacrylate,15.2g of Methacrylic Acid, and 57.3g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 121.6g of i-Butyl Methacrylate, 60.8g of Methacrylic Acid, and 229.3g of t-Octylacrylamide as a 60% solution in Ethanol was added over 3.5 hours using a 1000 mL addition funnel. After this addition was completed, the funnel was rinsed with 4.0g of Ethanol, which was then added to the reaction. 2 hours and 15 minutes after the start of reflux, 1.1g of Benzoyl Peroxide dissolved in 84.0g of i-Propyl Acetate was added over 2 hours. After this addition was completed the funnel was rinsed with 4.0g of i-Propyl Acetate which was then added to the reaction. The reaction was then allowed to reflux for an additional 8.5 hours. The reaction was then diluted with 108.4g of i-Propyl Acetate and 25.2g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 9

To a 2000mL reaction vessel equipped with an agitator, a heating mantle, a condenser, a 125 mL addition funnel, a 1000mL addition funnel, and a nitrogen purge was added 60.6g of i-Propyl Acetate. The stirring was turned on at a rate of 200 rpm, to the reaction was added 2.5g of Benzoyl Peroxide, 19.6g of i-Butyl Methacrylate, 15.2g of Methacrylic Acid, and 75.3g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 78.4g of i-Butyl Methacrylate, 60.8g of Methacrylic Acid, and 301.3g of t-Octylacrylamide as a 60% solution in Ethanol was added over 3.5 hours using a 1000 mL addition funnel. After this addition was completed, the funnel was rinsed with 4.0g of Ethanol, which was then added to the reaction. 2 hours and 15 minutes after the start of reflux, 1.1g of Benzoyl Peroxide dissolved in 84.0g of i-Propyl Acetate was added over 2 hours. After this addition was completed the funnel was rinsed with 4.0g of i-Propyl Acetate which was then added to the reaction. The reaction was then allowed to reflux for an additional 8.5 hours. The reaction was then diluted with 108.4g of i-Propyl Acetate and 25.2g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 10

To a 2000mL reaction vessel equipped with an agitator, a heating mantle, a condenser, a 125 mL addition funnel, a 1000mL addition funnel, and a nitrogen purge was added 60.6g of i-Propyl Acetate. The stirring was turned on at a rate of 200 rpm, to the reaction was added 2.5g of Benzoyl Peroxide, 23.6g of i-Butyl Methacrylate, 15.2g of Methacrylic Acid, and 68.7g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 94.4g of i-Butyl Methacrylate, 60.8g of Methacrylic Acid, and 274.8g of t-Octylacrylamide as a 60% solution in Ethanol was added over 3.5 hours using a 1000 mL addition funnel. After this addition was completed, the funnel was rinsed with 4.0g of Ethanol, which was then added to the reaction. 2 hours and 15 minutes after the start of reflux, 1.1g of Benzoyl Peroxide dissolved in 84.0g of i-Propyl Acetate was added over 2 hours. After this addition was completed the funnel was rinsed with 4.0g of i-Propyl Acetate which was then added to the reaction. The reaction was then allowed to reflux for an additional 8.5 hours. The reaction was then diluted with 108.4g of i-Propyl Acetate and 25.2g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 11 - not in accordance with the present invention

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 25.8g of i-Butyl Methacrylate, 14.4g of Methacrylic Acid, and 19.8g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 146.2g of i-Butyl Methacrylate, 81.6g of Methacrylic Acid, and 112.2g of t-Octylacrylamide as a 60% solution in Ethanol was added over 3.5 hours using a 1000 mL addition funnel. After this addition was completed, the funnel was rinsed with 5,0g of Ethanol, which was then added to the reaction. 2 hours and 15 minutes after the start of reflux, 1.2g of Benzoyl Peroxide dissolved in 84.6g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 65.2g of i-Propyl Acetate and 70.2g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 12 - not in accordance with the present invention

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 7.2g of Methacrylic Acid, and 88.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 40.8g of Methacrylic Acid, and 498.7g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 13

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 15.0g of Methacrylic Acid, and 75.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 85.0g of Methacrylic Acid, and 425.0g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 14 - not in accordance with the present invention

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 36.0g of i-Butyl Methacrylate, 9.6g of Methacrylic Acid, and 42.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 204.0g of i-Butyl Methacrylate, 54.4g of Methacrylic Acid, and 238.0g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 15

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 36.0g of i-Butyl Methacrylate, 12.6g of Methacrylic Acid, and 19.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 204.0g of i-Butyl Methacrylate, 71.4g of Methacrylic Acid, and 107.7g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 16

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 13.8g of Methacrylic Acid, and 77.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 78.2g of Methacrylic Acid, and 436.3g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 17

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 24.0g of i-Butyl Methacrylate, 12.6g of Acrylic Acid, and 39.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 136.0g of i-Butyl Methacrylate, 71.4g of Acrylic Acid, and 221,0g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 18 - not in accordance with the present invention

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 24.0g of i-Butyl Methacrylate, 9.6g of Acrylic Acid, and 44.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 136.0g of i-Butyl Methacrylate, 54.4g of Acrylic Acid, and 249.3g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 19 - not in accordance with the present invention

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 9.6g of Acrylic Acid, and 84.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 54.4g of Acrylic Acid, and 476.0g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 20

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 12.6g of i-Butyl Methacrylate, 11.1g of Acrylic Acid, and 61.5g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 71.4g of i-Butyl Methacrylate, 62.9g of Acrylic Acid, and 348.5g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

EXAMPLE 21

To a 2000mL reaction vessel equipped with an agitator, a heating mantle, a condenser, a 125 mL addition funnel, a 1000mL addition funnel, and a nitrogen purge was added 129.0g of i-Propyl Acetate. The stirring was turned on at a rate of 200 rpm, to the reaction was added 2.4g of Benzoyl Peroxide, 12.6g of Acrylic Acid, and 79.0g of t-Octylacrylamide as a 60% solution in Ethanol. The reaction was purged with Nitrogen for 15 minutes. The reaction was heated up to reflux, and then the Nitrogen purge was stopped. The reflux was maintained through the course of the reaction. 15 minutes after the start of reflux, a mixture of 71.4g of Acrylic Acid, and 447.7g of t-Octylacrylamide as a 60% solution in Ethanol was added over 3.5 hours using a 1000 mL addition funnel. After this addition was completed, the funnel was rinsed with 5.0g of Ethanol, which was then added to the reaction. 2 hours and 15 minutes after the start of reflux, 1.2g of Benzoyl Peroxide dissolved in 85.4g of i-Propyl Acetate was added over 2 hours. After this addition was completed the funnel was rinsed with 5.0g of i-Propyl Acetate which was then added to the reaction. The reaction was then allowed to reflux for an additional 8.5 hours. The reaction was then diluted with 70.9g of i-Propyl Acetate and 65.9g of Ethanol. The reaction was allowed to reflux an additional 45 minutes, and then cooled to room temperature. The polymer was isolated by making a thin film of the polymer i-Propyl Acetate/Ethanol solution. The film was allows to dry at room temperature overnight, then in an oven at 130°C for 90 minutes. The resulting dry film was then ground up in a blender to isolate the product as a powder.

Compositions of the polymers in examples 1 to 21 is summarized in Table 1. The weight % is based on the dry weight of the polymer.

TABLE 1

| Polymer Composition wt.% on a dry basis | | | | | |
|---|---|---|---|---|---|
| Example | %i-Butyl Methacrylate | %Methacrylic Acid | %Acrylic Acid | %t-Octylacrylamide | Acidity meq/g |
| 1 | 40.0 | 21.0 | 0.0 | 39.0 | 2.44 |
| 2* | 40.0 | 16.0 | 0.0 | 44.0 | 1.86 |
| 3* | 0.0 | 16.0 | 0.0 | 84.0 | 1.86 |
| 4 | 20.8 | 18.3 | 0.0 | 60.9 | 2.13 |
| 5 | 0.0 | 21.0 | 0.0 | 79.0 | 2.44 |
| 6 | 43.0 | 18.0 | 0.0 | 39.0 | 2.09 |
| 7 | 33.0 | 20.5 | 0.0 | 46.5 | 2.38 |
| 8 | 38.0 | 19.0 | 0.0 | 43.0 | 2.21 |
| 9 | 24.5 | 19.0 | 0.0 | 56.5 | 2.21 |
| 10 | 29.5 | 19.0 | 0.0 | 51.5 | 2.21 |
| 11* | 49.5 | 27.6 | 0.0 | 22.8 | 3.21 |

(continued)

| Polymer Composition wt.% on a dry basis | | | | | |
|---|---|---|---|---|---|
| Example | %i-Butyl Methacrylate | %Methacrylic Acid | %Acrylic Acid | %t-Octylacrylamide | Acidity meq/g |
| 12* | 0.0 | 12.0 | 0.0 | 88.0 | 1.39 |
| 13 | 0.0 | 25.0 | 0.0 | 75.0 | 2.90 |
| 14* | 50.8 | 13.6 | 0.0 | 35.6 | 1.58 |
| 15 | 60.0 | 21.0 | 0.0 | 19.0 | 2.44 |
| 16 | 0.0 | 23.0 | 0.0 | 77.0 | 2.67 |
| 17 | 40.0 | 0.0 | 21.0 | 39.0 | 2.91 |
| 18* | 40.0 | 0.0 | 16.0 | 44.0 | 2.22 |
| 19* | 0.0 | 0.0 | 16.0 | 84.0 | 2.22 |
| 20 | 20.8 | 0.0 | 18.3 | 60.9 | 2.54 |
| 21 | 0.0 | 0.0 | 21.0 | 79.0 | 2.91 |
| *Not in accordance with the present invention | | | | | |

Each of the 21 Examples and the commercial product AMPHOMER® (acidity 2.29meq/g) were made up at 5% polymer solids in 5 different solvent systems (100% ethanol, 100% i-Propanol, and 75/25/ i-Propanol/Ethanol, 80/20 Ethanol/Water, 80/20 i-Propanol/Water. The polymers were neutralized 100% with 2-amino-2-methyl-1-propanol.

The turbidity of the 5% polymer solutions neutralized 100% with 2-amino-2-methyl-1-propanol at 25°C was measured by placing 20 mLs in a HACH tube (glass tubes designed for the specific instrument). The turbidity was then measured on a HACH Turbidimeter (model number 2100N) and reported in NTU. Results are shown in Table 2.

The viscosity of the 5% polymer solutions neutralized 100% with 2-amino-2-methyl-1-propanol was measured at 50 rpm using an LVI Brookfield Digital Viscometer and a UL adapter at 25°C. The viscosity is reported in cps. Results are shown in Table 3.

TABLE 2

| Turbidity (ntus) of 5 %Polymer neutralized 100% with 2-Amino-2-methyl-1-Propanol in various solvent systems in | | | | | |
|---|---|---|---|---|---|
| Example | 100% Ethanol | 100% i-Propa | 75/25Ethanol/ i-Propanol | 80/20 Etha Water | 80/20 i-Propanol/ W |
| 1 | 1.08 | 2.80 | 1.48 | 2.19 | 1.82 |
| 2* | 0.99 | 1.53 | 1.28 | 23.10 | 25.6 |
| 3* | 1.48 | 2.58 | 1.86 | 295.0 | 320.0 |
| 4 | 1.10 | 3.35 | 1.42 | 72.4 | 62.6 |
| 5 | 4.40 | 9.40 | 5.27 | 132.0 | 121.0 |
| 6 | 0.87 | 2.35 | 1.23 | 2.23 | 1.35 |
| 7 | 1.00 | 2.65 | 1.01 | 34.9 | 31.6 |
| 8 | 2.09 | 2.50 | 1.92 | 11.6 | 9.18 |
| 9 | 2.14 | 3.16 | 2.06 | 38.1 | 2.55 |
| 10 | 1.87 | 3.43 | 1.74 | 84.6 | 1.83 |
| 11* | 1.93 | 49.40 | 1.65 | 1.77 | 4.03 |
| 12* | 2.58 | 3.97 | 2.94 | 871.0 | 1.83 |
| 13 | 2.47 | 121.00 | 2.90 | 96.3 | 2.17 |
| 14* | 1.68 | 2.50 | 1.48 | 1.17 | 4.17 |

(continued)

| Turbidity (ntus) of 5 %Polymer neutralized 100% with 2-Amino-2-methyl-1-Propanol in various solvent systems in | | | | | |
|---|---|---|---|---|---|
| Example | 100% Ethanol | 100% i-Propa | 75/25Ethanol/ i-Propanol | 80/20 Etha Water | 80/20 i-Propanol/ W |
| 15 | 3.20 | 3.75 | 2.70 | 1.67 | 6.32 |
| 16 | 3.99 | 101.00 | 3.85 | 56.5 | 1.61 |
| 17 | 1.08 | 1.22 | 1.13 | 1.54 | 2.40 |
| 18* | 1.35 | 1.19 | 1.66 | 1.90 | 2.00 |
| 19* | 1.61 | 1.81 | 1.39 | 1.32 | 1.54 |
| 20 | 1.50 | 1.40 | 1.46 | 1.14 | 3.29 |
| 21 | 1.96 | 2.24 | 2.13 | 1.08 | 1.87 |
| AMPHOMER | 1.72 | 20.00 | 1.63 | 2.21 | 1.49 |
| *Not in accordance with the present invention | | | | | |

TABLE 3

| Viscosity (cps) of 5 %Polymer neutralized 100% with 2-Amino-2-methyl-1-Propan various solvent systems ol in | | | | | |
|---|---|---|---|---|---|
| Example | 100% Ethanol | 100% i-Propa | 75/25Ethanol/ i-Propanol | 80/20 Etha Water | 80/20 i-Propanol/ W |
| 1 | 5.38 | 8.32 | 5.12 | 10.3 | 10.5 |
| 2* | 4.22 | 6.40 | 5.76 | 7.36 | 7.42 |
| 3* | 2.56 | 3.84 | 2.82 | 4.22 | 4.56 |
| 4 | 3.71 | 5.63 | 3.84 | 6.47 | 6.53 |
| 5 | 3.20 | 4.74 | 4.48 | 5.52 | 5.32 |
| 6 | 5.32 | 7.49 | 5.12 | 9.54 | 8.64 |
| 7 | 4.29 | 6.85 | 4.55 | 8.70 | 8.77 |
| 8 | 6.13 | 8.94 | 6.37 | 8.52 | 9.73 |
| 9 | 4.74 | 6.21 | 3.97 | 7.55 | 11.7 |
| 10 | 3.91 | 6.34 | 4.35 | 6.97 | 10.2 |
| 11* | 6.57 | 12.00 | 6.85 | 12.0 | 13.4 |
| 12* | 2.50 | 3.78 | 2.50 | 3.33 | 5.06 |
| 13 | 3.01 | 4.68 | 3.40 | 6.34 | 8.26 |
| 14* | 4.16 | 6.53 | 3.91 | 6.34 | 8.77 |
| 15 | 4.29 | 6.47 | 4.79 | 4.89 | 6.32 |
| 16 | 3.46 | 4.61 | 2.94 | 5.44 | 7.04 |
| 17 | 5.19 | 3.46 | 2.18 | 2.94 | 2.24 |
| 18* | 4.29 | 3.46 | 2.05 | 3.01 | 2.24 |
| 19* | 4.22 | 3.27 | 2.05 | 2.88 | 2.18 |
| 20 | 9.92 | 6.97 | 3.48 | 5.57 | 3.91 |
| 21 | 11.30 | 8.96 | 4.93 | 7.81 | 5.96 |

(continued)

| Viscosity (cps) of 5 %Polymer neutralized 100% with 2-Amino-2-methyl-1-Propan various solvent systems ol in | | | | | |
|---|---|---|---|---|---|
| Example | 100% Ethanol | 100% i-Propa | 75/25Ethanol/ i-Propanol | 80/20 Etha Water | 80/20 i-Propanol/ W |
| AMPHOMER | 5.12 | 8.32 | 5.76 | 12.5 | 13.4 |
| *Not in accordance with the present invention | | | | | |

**Determination of Mean Particle Size**

Polymers 1-21 in various solvent systems were tested in order to determine the mean particle size of the sample formulations. Particle Size was measured on the formulations as delivered from the aerosol can or pump spray. Particle Size was measured on a Malvern Particle Size Analyzer Spraytec 2600 droplet and particle size analyzer. Products was positioned 8-10" from the laser. Products are actuated and the instrument performs the measurements and calculations.

Particle size is a key performance property to consider when developing a hairspray formulation. Particle size is a combination of the solubility of the polymer in the solvents, the viscosity of the solvent, and the surface tension of the solvent. The capillary number marries all three of these properties together to predict particle size. If the spray is too small it dries before it hits the hair creating no hold. If the particles are too large the sprays take too long to dry and weigh the hair down. The results of the mean particle size (D[3][2]) measurements are shown in Table 4.

Aerosols using 100% i-Propanol as the solvent were made up with the polymer at 5% solids neutralized 100% 2-Amino-2-Methyl-1-Propanol and 40%1,1-Difluoroethane. i-Propanol was present to add up to 100%. Aerosols using 80/20 i-Propanol/Water as the solvent were made up with the polymer at 5% solids neutralized 100% 2-Amino-2-Methyl-1-Propanol and 40% Dimethyl Ether. 80/20 i-Propanol/Water was present to add up to 100%.

TABLE 4

| Example | 100% i-Propanol | 80/20 i-Propanol/ Water |
|---|---|---|
| 1 | 109.4 | 50.9 |
| 2* | 85.9 | 43.7 |
| 3* | 37.9 | did not run |
| 4 | 72.7 | did not run |
| 5 | 46.6 | did not run |
| 6 | 128.0 | 55.4 |
| 7 | 124.0 | 54.6 |
| 8 | 129.4 | 57.8 |
| 9 | 80.6 | 41.3 |
| 10 | 88.9 | 48.0 |
| 11* | would not spray | 89.8 |
| 12* | 38.9 | 33.0 |
| 13 | 46.5 | 39.0 |
| 14* | 74.2 | 41.5 |
| 15 | 122.2 | 66.7 |
| 16 | 46.9 | 35.4 |
| 17 | 67.1 | 54.6 |
| 18* | 53.4 | 42.8 |
| 19* | 34.6 | 35.3 |
| 20 | 45.5 | 36.1 |

(continued)

| Example | 100% i-Propanol | 80/20 i-Propanol/ Water |
|---------|-----------------|-------------------------|
| 21 | 35.0 | 36.5 |
| AMPHOMER | 153.7 | 65.2 |
| *Not in accordance with the present invention | | |

## Determination of High Humidity Curl Retention (HHCR)

Aerosols using 80/20 i-Propanol/Water as the solvent were made up with the polymer at 5% solids neutralized 100% 2-Amino-2-Methyl-1-Propanol and 40% Dimethyl Ether. 80/20 i-Propanol/Water was present to add up to 100%. The high humidity curl retention properties of hair styling compositions of the present invention were measured. The test was conducted at 72°F (22°C) and 90% Relative Humidity over a period of 24 hours. The test was performed on 10" long x 2-gram swatches of European virgin brown hair (9 replicate swatches per sample). Curl retention testing is run in a humidity chamber set at 70°F/90% Relative Humidity for a total of 24 hours. Readings for % Curl Retention are read and recorded at time intervals of 15, 30, 60, 90 min, 2, 3, 4, 5, and 24 hrs. The hair styling compositions were tested according to the following procedures:

1. Wet hair swatch, comb through to remove tangles and squeeze out excess water (run swatch between thumb and index finger).
2. Apply sample to swatch, gently "work into" swatch and comb through.
3. Roll swatch on 1/2" diameter Teflon mandrel. Carefully remove rolled swatch from mandrel and secure with two hair clips.
4. Place curls on tray and dry in oven overnight.
5. Remove dried curls from oven and let cool to room temperature.
6. Suspend curls, from bound end of swatch, on graduated clear, transparent curl retention boards.
7. Remove clips from curls and gently unwind with glass rod making sure to "break" the curl.
8. Take initial curl length readings before placing boards and curls into environmental chamber (70°F, 90% relative humidity).
9. Record curl lengths at the 15, 30, 60, 90, 2, 3, 4, 5, and hour time intervals.
10. At conclusion of test, remove boards and curls from chamber.
11. Clean used hair swatches.
12. Calculate % Curl Retention and comparison of samples.

The Samples were prepared as follows:
HHCR was run in a constant temperature and humidity chamber. Curls were rolled on a mandrel and allowed to dry overnight. The curls were then sprayed with the polymer solutions and allowed to dry. Then the curls were hung on a board placed in the oven and the percent of curl loss was tracked over 24 hrs.

TABLE 5

| Example | % Curl Retention after 24 Hours |
|---------|--------------------------------|
| 1 | 94.1 |
| 2* | 96.7 |
| 3* | did not run |
| 4 | did not run |
| 5 | did not run |
| 6 | 97.1 |
| 7 | 95.0 |
| 8 | 94.8 |
| 9 | 97.0 |
| 10 | 96.1 |

(continued)

| Example | % Curl Retention after 24 Hours |
|---|---|
| 11* | 94.0 |
| 12* | 100.0 |
| 13 | 98.5 |
| 14* | 100.0 |
| 15 | 98.7 |
| 16 | 96.3 |
| 17 | 95.0 |
| 18* | 95.1 |
| 19* | 95.9 |
| 20 | 95.1 |
| 21 | 97.3 |
| AMPHOMER | 95.2 |
| *Not in accordance with the present invention | |

As shown in Table 5, all of the polymers tested gave high levels of High Humidity Curl Retention after 24 hours.

**Claims**

1. A hair fixative composition comprising:

   at least one fixative polymer wherein the polymer is derived from 18 to 25 weight percent of at least one acid-containing monomer, based on the total weight of the monomer content in the polymer, and at least one N-alkyl (meth)acrylamide that is N-n-octyl acrylamide or N-t-octyl acrylamide; and
   a solvent system comprising one or more solvents selected from the group consisting of a $C_2$-$C_6$ straight or branched chain alcohol, butyl cellusolve, propylene glycol, water and mixtures thereof,
   with the proviso that the at least one fixative polymer is solution polymerized such that the fixative polymer is a solute dissolved in the solvent system.

2. The hair fixative composition of claim 1 wherein the at least one acid-containing monomer is selected from the group consisting of methacrylic acid, acrylic acid, maleic acid, fumaric acid, itaconic acid and mixtures thereof.

3. The hair fixative composition of claims 1 or 2 wherein the at least one fixative polymer further comprises at least one alkyl (meth)acrylate monomer.

4. The hair fixative composition of claims 1 to 3 wherein the at least one fixative polymer is present in an amount of 0.25 to 8.0 weight percent of the composition.

5. The hair fixative composition of claims 1 to 4 wherein the composition further comprises a propellant.

6. The hair fixative composition of claim 1 comprising:
   5 to 30 weight percent or 60 to 90 weight percent of the at least one N-alkyl (meth)acrylamide that is N-n-octyl acrylamide or N-t-octyl acrylamide, based on the total weight of the monomer content in the polymer.

**Patentansprüche**

1. Haarfestigerzusammensetzung mit:

mindestens einem Festigerpolymer, wobei das Polymer aus 18 bis 25 Gewichtsprozent von mindestens einem säurehaltigen Monomer, bezogen auf das Gesamtgewicht des Monomergehaltes in dem Polymer, und mindestens einem N-Alkyl-(Meth)acrylamid, nämlich N-n-Octylacrylamid oder N-t-Octylacrylamid, abgeleitet ist; sowie

einem Lösungsmittelaufbau mit einem oder mehreren Lösungsmitteln ausgewählt aus der Gruppe bestehend aus einem $C_2$-$C_6$ gerad- oder verzweigtkettigen Alkohol, Butyl-Cellosolve, Propylenglykol, Wasser sowie Mischungen aus diesen,

mit der Maßgabe, dass das mindestens eine Festigerpolymer lösungspolymerisiert ist, so dass das Festigerpolymer aus einem gelösten Stoff besteht, der in dem Lösungsmittelaufbau aufgelöst ist.

2. Haarfestigerzusammensetzung nach Anspruch 1, wobei das mindestens eine säurehaltige Monomer aus der Gruppe bestehend aus Methacrylsäure, Acrylsäure, Maleinsäure, Fumarsäure, Itakonsäure sowie Mischungen aus diesen ausgewählt ist.

3. Haarfestigerzusammensetzung nach Anspruch 1 oder 2, wobei das mindestens eine Festigerpolymer weiterhin mindestens ein Alkyl-(Meth)acrylatmonomer enthält.

4. Haarfestigerzusammensetzung nach Anspruch 1 bis 3, wobei das mindestens eine Festigerpolymer in einer Menge von 0,25 bis 8,0 Gewichtsprozent der Zusammensetzung vorliegt.

5. Haarfestigerzusammensetzung nach Anspruch 1 bis 4, wobei die Zusammensetzung weiterhin ein Treibmittel enthält.

6. Haarfestigerzusammensetzung nach Anspruch 1, mit:
5 bis 30 Gewichtsprozent oder 60 bis 90 Gewichtsprozent des mindestens einen N-Alkyl-(Meth)acrylamid, nämlich N-n-Octylacrylamid oder N-t-Octylacrylamid, bezogen auf das Gesamtgewicht des Monomergehaltes in dem Polymer.

## Revendications

1. Composition de fixateur pour cheveux comprenant :

au moins un polymère fixateur où le polymère est dérivé de 18 à 25 pour cent en poids d'au moins un monomère contenant un acide, par rapport au poids total de la teneur en monomère dans le polymère, et d'au moins un N-alkyl(méth)acrylamide qui est le N-n-octylacrylamide ou le N-t-octylacrylamide ; et
un système de solvant comprenant un ou plusieurs solvant(s) choisi(s) dans le groupe constitué d'un alcool à chaîne droite ou ramifiée en $C_2$ à $C_6$, de butyl cellusolve, de propylèneglycol, d'eau et de mélanges de ceux-ci, à condition que l'au moins un polymère fixateur soit polymérisé en solution de sorte que le polymère fixateur soit un soluté dissous dans le système de solvant.

2. Composition de fixateur pour cheveux de la revendication 1, dans laquelle l'au moins un monomère contenant un acide est choisi dans le groupe constitué par l'acide méthacrylique, l'acide acrylique, l'acide maléique, l'acide fumarique, l'acide itaconique et des mélanges de ceux-ci.

3. Composition de fixateur pour cheveux de la revendication 1 ou 2, dans laquelle l'au moins un polymère fixateur comprend en outre au moins un monomère de (méth)acrylate d'alkyle.

4. Composition de fixateur pour cheveux des revendications 1 à 3, dans laquelle l'au moins un polymère fixateur est présent en une quantité allant de 0,25 à 8,0 pour cent en poids de la composition.

5. Composition de fixateur pour cheveux des revendications 1 à 4, dans laquelle la composition comprend en outre un agent propulseur.

6. Composition de fixateur pour cheveux de la revendication 1 comprenant :
5 à 30 pour cent en poids ou 60 à 90 pour cent en poids de l'au moins un N-alkyl(méth)acrylamide qui est le N-n-octylacrylamide ou le N-t-octylacrylamide, par rapport au poids total de la teneur en monomère dans le polymère.